(19) Europäisches Patentamt

European Patent Office

Office européen des brevets

(11) EP 0 723 766 A1

(12) EUROPEAN PATENT APPLICATION

(43) Date of publication:
31.07.1996 Bulletin 1996/31

(51) Int. Cl.$^6$: A61F 2/06

(21) Application number: 96200184.8

(22) Date of filing: 26.01.1996

(84) Designated Contracting States:
AT BE CH DE DK ES FR GB IE IT LI NL PT SE

(30) Priority: 26.01.1995 NL 9500147

(71) Applicant: CORDIS EUROPA N.V.
NL-9301 LJ Roden (NL)

(72) Inventor: Glastra, Hendrik
NL-7535 PG Enschede (NL)

(74) Representative: 't Jong, Bastiaan Jacobus
Arnold & Siedsma,
Advocaten en Octrooigemachtigden,
Sweelinckplein 1
2517 GK Den Haag (NL)

(54) **Method for manufacturing a sleeve-formed stent from foil material, and stent obtained with said method**

(57) Method for manufacturing from foil material a sleeve-formed stent filled with curable material, according to which a single-walled basic sleeve is folded back into itself so as to form a double-walled sleeve portion, curable material is arranged in the space between both walls of the double-walled sleeve portion, and the extending single-walled sleeve portion is folded back over the outer wall of the double-walled sleeve portion and connected thereto.

FIG. 3d.

EP 0 723 766 A1

## Description

The invention relates to a method for manufacturing from foil material a double-walled sleeve formed stent closed at both ends having curable material enclosed between the walls.

Such a stent and a method for manufacturing thereof are described in EP-A-0 617 930 in the name of applicant.

The invention has for its object to improve the known method in such a way that it is specifically suitable for industrial application, so that stents of different sizes can be realized swiftly and accurately.

According to the invention this object is achieved by a method comprising the steps of:

- forming from foil material single-walled basic sleeve with a diameter related to the diameter of the stent to be formed,
- folding back into itself a portion of this single sleeve so as to form a double-walled sleeve portion wherein the distance between both walls lying at a distance relative to one another is related to the length of the stent to be formed, and having a single-walled sleeve portion adjoining thereto,
- arranging curable material in the space between both walls of the double-walled sleeve portion,
- folding back the single-walled sleeve portion extending beyond the end of the double-walled sleeve portion over the outer wall of the double-walled sleeve portion and connecting said folded back portion to said outer wall.

The method steps according to the invention can be performed mechanically by means of simple auxiliary tools so that the object of the invention: rationally manufacturing stents - can be realized.

The method according to the invention is preferably performed in the way described in claims 2-6.

The right applied for extends also to a stent obtained by using the method as described above.

A special effect of the method according to the invention is that it is possible herewith to realize stents of larger sizes in a rational manner. Such stents have the advantage that it is possible to support therewith a body vessel, for example a blood vessel, over a larger extent (e.g. 10 to 15 cm). Moreover, the desired local stiffness of the stent can be varied by applying distributed over the length a more or less thick layer of curable material received in absorbing material.

The invention is illustrated by means of the drawing, in which:

Fig. 1        shows a longitudinal section of a stent known as such,

Fig. 2        a perspective view of the sleeve used with the method according to the invention,

Figs. 3a-3e   show schematically and in longitudinal section the several steps of the method according to the invention,

Figs. 4a-4b   show schematically and in longitudinal section two steps of an alternative of this method,

Figs. 5a-5c   show schematically a method for manufacturing the basic sleeve,

Fig. 6        is perspective representation of the basic sleeve obtained according to Figs. 5a-5c.

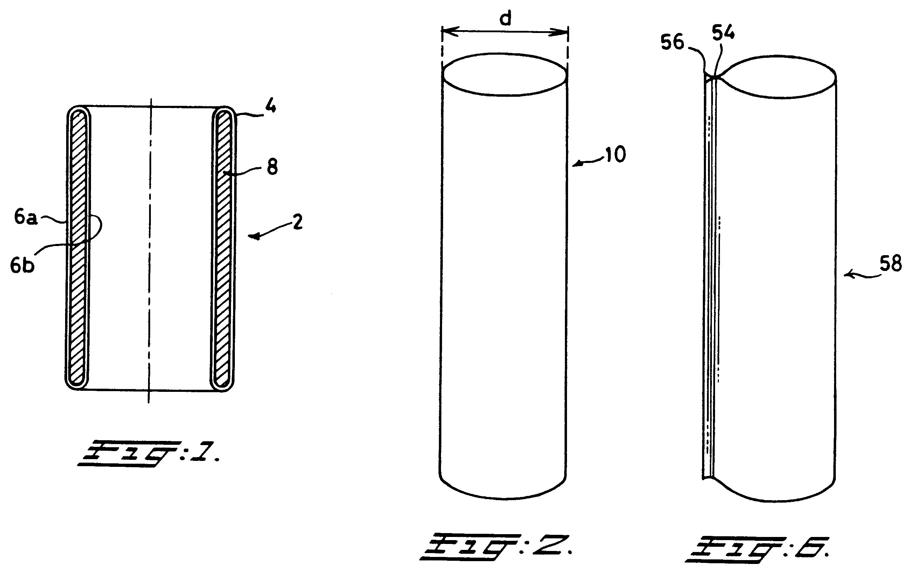

Fig. 1 shows a longitudinal section of a stent, indicated integrally with the reference numeral 2, of the kind known as such from EP-A-0 521 573 and EP-A-0 617 930, both in the name of applicant. The stent 2 essentially exists of an elongated double-walled sleeve 4 of suitable foil material, for example teflon, which is closed in itself. In the space between the walls 6a, 6b curable material 8 is arranged which preferable is received in mesh-like material in the way described in EP-A-0 617 930. As described in that publication such a stent is introduced through a catheter to the right position in a body vessel, by means of a carrier - and positioning balloon; subsequently the balloon is expanded by supplying thereto a medium under pressure whereby also the stent adopts the ultimate cylindrical form, while adapting to the wall profile of the vessel and subsequently the curable material is made to cure by irradiation with a suitable radiation, for example UV radiation. When the curing is finished, the stent has become dimensionally stable and is anchored fixedly in the body vessel.

EP-A-0 617 930 already describes a method for manufacturing such a stent; the invention has for its object to provide an improved method specifically suitable for large scale industrial manufacturing.

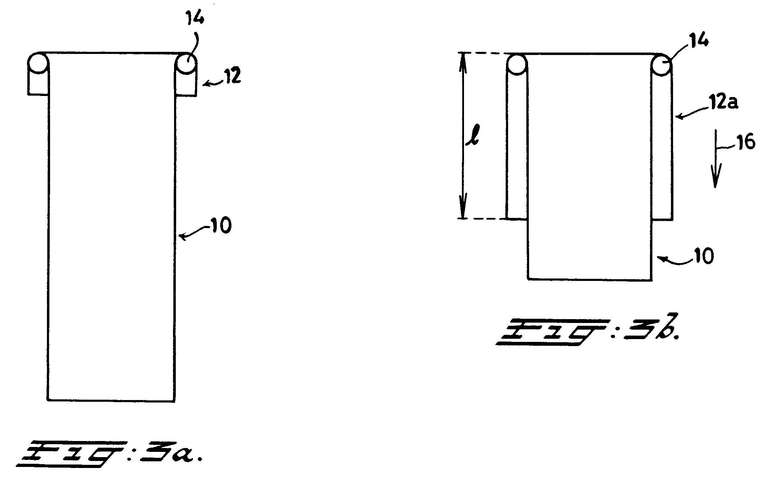

According to the invention starting point is a sleeve from foil material closed in itself as drawn in perspective in Fig. 2, and referenced therein with reference numeral 10. This sleeve 10 has a diameter d related to the diameter of the stent ultimately to be formed.

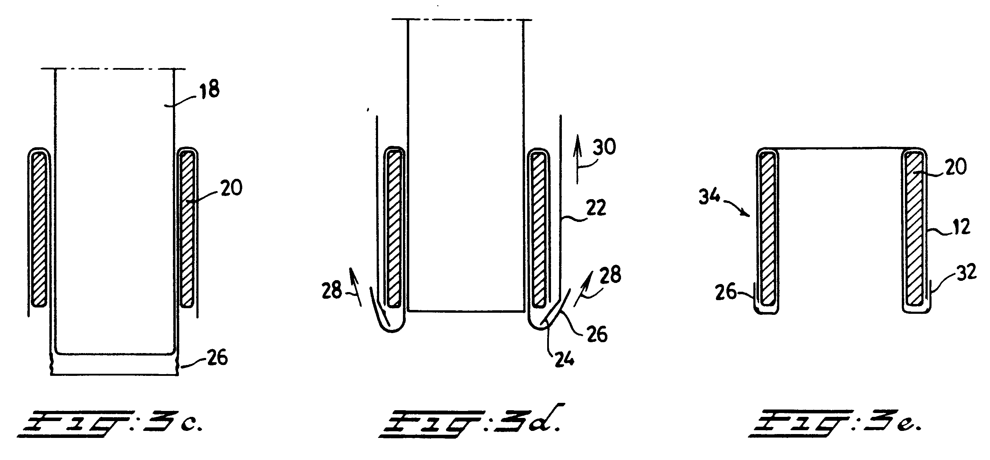

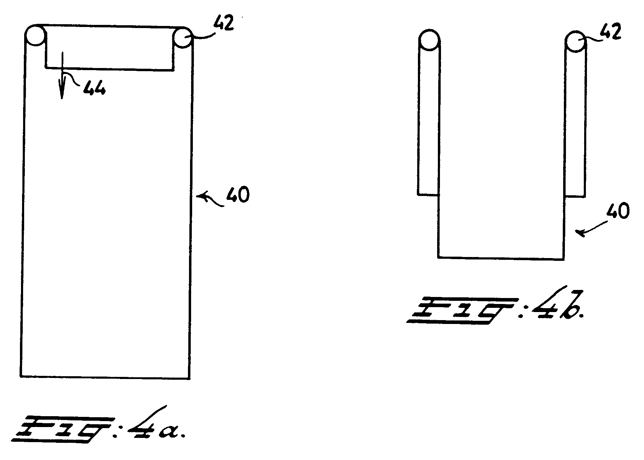

From the sleeve 10 first a double-walled sleeve is formed, for which the invention offers to possibilities which are illustrated by Figs. 3a-3e at the one hand and Figs. 4a-4b at the other hand.

Fig. 3a shows how sleeve 10 is folded outward with its end 12 over a circular guiding edge 14 and subsequently is pulled further by grippers not shown to the situation indicated with 12a in Fig. 3b, in the direction of arrow 16 over a distance l which is related to the length of the stent ultimately to be formed.

Subsequently, the guiding edge 14 is removed and the sleeve 10 which is now partly double-walled, is arranged on a mandrel 18 - see Fig. 3c - whereafter inside the double-walled portion the curable material 20 received in suitable mesh-like material, is arranged. Using a guiding member 22 with circular edge 24, which is arranged around the edge 26 of sleeve 10, this edge 26 is pulled upward by means of suitable grippers not shown, in the direction of arrows 28, meanwhile moving the guiding member 22 in the direction of arrow 30, until edge 26 closes around the outer wall 12a of the sleeve 10, until the situation shown in Fig. 3d is obtained. Finally, the edge 26 is fixed to the outer wall 12a at position 32 and the stent 34 is ready - see Fig. 3c.

Forming the double-walled sleeve is also possible, as Figs. 4a-4b show, by pulling a sleeve 40 from the outside to the inside over a cylindrical guiding edge 42 in the direction of arrows 44 to the configuration shown in Fig. 4b; thereafter the same steps are performed as described above.

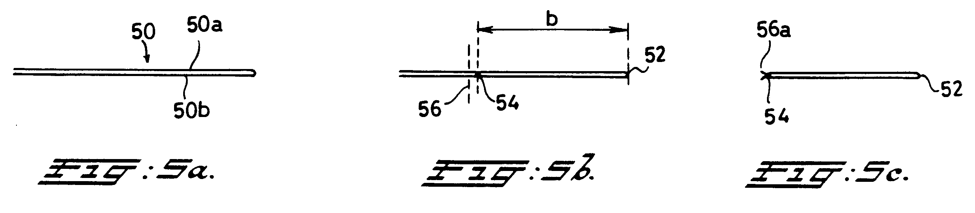

According to the invention the sleeve is preferably obtained by starting with a folded foil 50 according to Fig. 5a, both foil parts 50a, 50b lying onto each other are connected to one another by a continuous elongate weld 54, at a distance b of the fold 52, which distance b is related to the diameter of the stent ultimately to be manufactured ($2b = \pi D$). Subsequently, the foil material is cut at position 56, just beyond weld 54, resulting in the situation according to Fig. 5c and a sleeve 58 having a configuration as shown in Fig. 5. In practice, the edges 56a extending over a short distance appear to produce absolutely no hindrance.

It will be clear that the method as described above is specifically suitable for industrial manufacturing; the man skilled in the art will be able to design the necessary auxiliary elements (guiding parts, grippers, driving members, etc.).

## Claims

1. Method for manufacturing from foil material a double-walled sleeve-formed stent closed at both ends and having curable material enclosed between the walls, characterized by:

   - forming from foil material a single-walled basic sleeve with a diameter related to the diameter of the stent to be formed,
   - folding back into itself a portion of this single sleeve so as to form a double-walled sleeve portion wherein the distance between both spaced apart walls is related to the length of the stent to be formed, and with an adjoining single-walled sleeve portion,
   - arranging curable material in the space between both walls of the double-walled sleeve portion,
   - folding back the single-walled sleeve portion extending beyond the end of the double-walled sleeve portion over the outer wall of the double-walled sleeve portion and connecting said folded-back portion to said outer wall.

2. Method according to claim 1, characterized in that the step of folding back the extending single sleeve portion is executed while using a guiding member enclosing the double-walled sleeve portion and being movable relative thereto.

3. Method according to claims 1-2, characterized in that the step of folding back into itself a portion of the single sleeve is executed while using a guiding edge enclosing said sleeve and pulling the single sleeve outwardly over said edge, over a distance related to the length of the stent to be formed.

4. Method according to claims 1-2, characterized in that the step of folding back into itself of the single sleeve is executed while using a guiding edge situated within said sleeve and pulling the single sleeve inwardly over said edge, over a distance related to the length of the stent to be formed.

5. Method according to claims 1-4, characterized in that the single sleeve portion is formed by:

   - folding double a sheet of foil material,
   - welding together both sheet portions at a distance of the fold, determined by the desired sleeve diameter, according to a line parallel to the fold,
   - cutting the material along the weld at the side facing away from the fold.

6. Method according to claims 1-5, characterized in that the curable material arranged in absorbent material is supplied over the length in a more or less thick layer.

7. Stent obtained with the method according to claims 1-6.

Fig.6.

Fig.2.

Fig.1.

Fig. 3b.

Fig. 3a.

FIG:3c.

FIG:3d.

FIG:3e.

EP 0 723 766 A1

Fig. 4b.

Fig. 4a.

Fig.5c.

Fig.5b.

Fig.5a.

EP 0 723 766 A1

European Patent
Office

EUROPEAN SEARCH REPORT

Application Number
EP 96 20 0184

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| D,A | EP-A-0 617 930 (INDUSTRIAL RESEARCH B.V.) 5 October 1994 * the whole document * | 1 | A61F2/06 B29C67/00 |

TECHNICAL FIELDS
SEARCHED      (Int.Cl.6)

A61F
B29C

The present search report has been drawn up for all claims

| Place of search | Date of completion of the search | Examiner |
|---|---|---|
| THE HAGUE | 14 May 1996 | Steenbakker, J |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

9